Europäisches Patentamt

European Patent Office

Office européen des brevets

(19)

(11) Numéro de publication: **0 168 315**
**B1**

(12) **FASCICULE DE BREVET EUROPEEN**

(45) Date de publication du fascicule du brevet:
**24.08.88**

(21) Numéro de dépôt: **85401324.0**

(22) Date de dépôt: **28.06.85**

(51) Int. Cl.⁴: **C 07 C 31/26**

(54) Procédé de préparation de sirops de sorbitol de très haute pureté.

(30) Priorité: **29.06.84 FR 8410256**

(43) Date de publication de la demande:
**15.01.86 Bulletin 86/3**

(45) Mention de la délivrance du brevet:
**24.08.88 Bulletin 88/34**

(84) Etats contractants désignés:
**AT BE CH DE GB IT LI NL**

(56) Documents cités:
**FR - A - 1 422 060**
**FR - A - 2 504 523**
**US - A - 4 442 881**

(73) Titulaire: **Roquette Frères, F-62136 Lestrem (FR)**

(72) Inventeur: **Devos, Francis, 70, rue de Merville La Motte-au-Bois/Morbecque, F-59190 Hazebrouck (FR)**
Inventeur: **Huchette, Michel, 63, rue du Maréchal Joffre, F-59660 Merville (FR)**

(74) Mandataire: **Koch, Gustave et al, Cabinet PLASSERAUD 84, rue d'Amsterdam, F-75009 Paris (FR)**

ACTORUM AG

### Description

L'invention a pour objet un procédé de préparation de sirops de sorbitol de très haute pureté.

Actuellement, les sirops de sorbitol de très haute pureté sont obtenus par l'hydrogénation de sirops de dextrose préparés par dissolution dans l'eau de dextrose cristallisé.

La richesse des sirops de sorbitol ainsi obtenus qui est suffisante, excède toutefois rarement 98% après purification.

La raison en est qu'en plus des traces de polyholosides hydrogénés provenant de la matière première et des traces de substances réductrices ayant échappé à l'hydrogénation, il se forme pendant l'hydrogénation, des isomères du sorbitol, à savoir notamment du mannitol. La présence de cet ensemble de produits entraîne certains inconvénients sur le plan des opérations de cristallisation du sorbitol à partir de ces sirops et a des conséquences sur les caractéristiques du sorbitol cristallisé obtenu, notamment du point de vue de sa cristallinité.

De plus, le rendement de l'opération n'est pas entièrement satisfaisant car, pour arriver aux sirops de sorbitol présentant cette richesse qui, de toute façon, excède rarement 98%, il y a lieu de s'accommoder de rendements limités au niveau de la préparation préalable du dextrose cristallisé, qui sert à la préparation du sirop de dextrose subséquemment hydrogéné, et qui est obtenu par cristallisation en deux ou trois jets à partir d'hydrolysats d'amidon d'une teneur en glucose de l'ordre de 94 à 96% en poids; en effet, lesdits rendements en dextrose sont généralement limités à environ 75–77% en deux jets et à environ 80–88% après un troisième jet et les eauxmères de cristallisation ou hydrols ont un écoulement parfois difficile sur le marché en raison de leur coloration et de leur teneur en impuretés.

Il a bien déjà été proposé de supprimer l'étape intermédiaire de cristallisation du dextrose en soumettant directement, à l'intérieur d'une même enceinte réactionnelle, une matière première bon marché, à savoir un hydrolysat d'amidon, non seulement à des conditions réductrices, mais encore à une hydrolyse au moyen d'un acide fort, ce qui permet de plus de tirer profit de la transformation en elle-même connue, des polyholosides tels que le maltitol ou l'isomaltitol en quantités équimoléculaires de sorbitol et de glucose sous l'effet de l'acide fort.

Il est possible de citer à cet égard le brevet français N° 1 263 298 qui propose l'utilisation, à titre de catalyseur, du nickel réduit supporté par une terre d'infusoires et suivant lequel on réalise dans une seule et même enceinte réactionnelle, en solution sensiblement neutre et à une température modérément élevée, une réduction de la majeure partie des sucres réducteurs, après quoi on ajoute un acide minéral fort tel que l'acide phosphorique, dans une proportion de 0,05 à 1,0% en poids sur la base des saccharides initialement mis en œuvre, le mélange constitué par la solution ainsi acidifiée et le catalyseur étant soumis à une pression et une température plus élevées, ce grâce à quoi sont réalisées simultanément l'hydrolyse des polyholosides hydrogénés présents dans l'hydrolysat et l'hydrogénation des sucres apparaissant alors.

Dans le même ordre d'idées, mais une quinzaine d'années plus tard, le brevet belge N° 837 201 décrit l'hydrogénation-hydrolyse-hydrogénation simultanées d'hydrolysats d'amidon avec recours à un catalyseur au ruthénium supporté sur zéolithe du type Y; la réaction d'hydrogénation est effectuée en deux étapes, la première étant conduite à une température comprise entre 100 et 175 °C et la seconde à une température d'environ 170 à 200 °C. Les conditions de pH dans lesquelles se déroulent les phases consécutives de l'hydrogénation sont précisées, le pH du produit de la réaction devant être compris entre 3,5 et 4.

Les inconvénients de ces procédés résident non seulement dans la rapide désactivation des catalyseurs et dans la dénaturation de la structure des zéolithes, mais également dans le fait que les sirops ainsi obtenus comprennent de nombreuses impuretés.

Ainsi,

– il se produit des réactions d'anhydrisation partielle et d'isomérisation des polyols,

– l'hydrolyse de la fraction de polyholosides hydrogénés est incomplète et se traduit par une valeur de «sucres totaux» élevée,

– les proportions d'«hexitannes», de mannitol, d'«impuretés non sucre totales» sont importantes, la teneur en sorbitol vrai étant finalement au plus égale à 94%, ce qui est par exemple très insuffisant pour la préparation d'un sorbitol cristallisé de qualité convenable.

Un autre procédé propre à la préparation de sirop de sorbitol sans passage par la cristallisation du dextrose est celui décrit dans le brevet français N° 2 052 202 déposé par la Société Demanderesse. Selon ce procédé, dans lequel on a également recours, en tant que matière première, à un hydrolysat d'amidon que l'on hydrogène, on réalise un fractionnement de l'hydrolysat d'amidon hydrogéné par passage sur une résine ou un tamis moléculaire cationique, de préférence sous forme calcium, ce qui permet d'isoler une fraction renfermant du sorbitol d'une richesse supérieure à 99%.

Cette technique conduit donc à un sorbitol d'une pureté très élevée, mais elle s'avère cependant non satisfaisante dans la pratique. Elle conduit en effet à un rendement très partiel en sorbitol, dépendant étroitement de la teneur en dextrose vrai de l'hydrolysat d'amidon de départ, celle-ci devant de ce fait être la plus élevée possible.

Il s'ensuit qu'a priori aucun des procédés connus ne permet à la fois de fabriquer du sirop de sorbitol de pureté élevée, convenant notamment à la préparation de sorbitol cristallisé, et d'offrir aisément un rendement élevé dont l'influence sur le prix de revient du produit final apparaît aisément.

C'est à ce problème que la Société Demanderesse a eu le mérite d'apporter une solution particulièrement efficiente, suivant laquelle une matière première dont une première partie est constituée par un hydrolysat d'amidon, est soumise à un procédé comprenant en combinaison

– une étape d'hydrogénation catalytique de la matière première,

– une étape de séparation chromatographique de la matière première ayant subi l'hydrogénation en une première fraction contenant du sorbitol très pur et qui est recupérée et en une deuxième fraction contenant, outre du sorbitol, des sucres non hydrogénés ainsi que des di- et polysaccharides hydrogénés,

– une étape d'hydrolyse acide de la susdite deuxième fraction, ce qui fournit un hydrolysat d'amidon partiellement hydrogéné qui est recyclé et qui constitue une deuxième partie de la matière première soumise à l'étape d'hydrogénation.

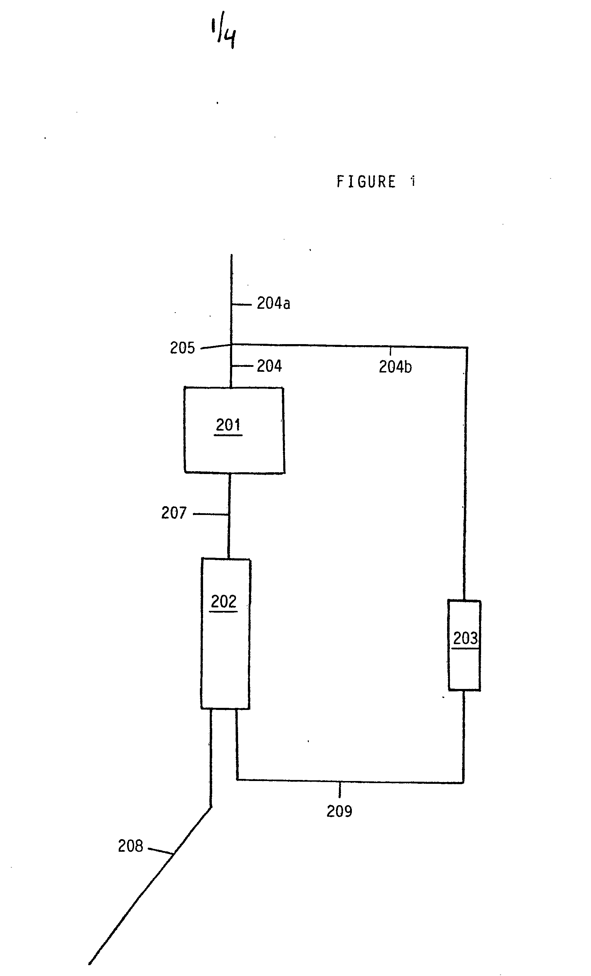

Le procédé ainsi défini peut être mis en œuvre à l'aide de l'installation schématiquement représentée à la figure 1 et qui comprend:

– une enceinte 201 à l'intérieur de laquelle est réalisée l'étape d'hydrogénation catalytique,

– une installation de séparation chromatographique 202 et

– une enceinte réactionnelle 203 à l'intérieur de laquelle est réalisée l'étape d'hydrolyse.

L'enceinte 201 est alimentée en matière première par une canalisation 204 formée par la réunion en 205 d'une canalisation 204a amenant l'hydrolysat d'amidon depuis un réservoir non montré et d'une canalisation 204b reliée à la sortie de l'enceinte 203 et amenant l'hydrolysat partiellement hydrogéné.

La sortie de l'enceinte 201 est reliée à l'entrée de l'enceinte 202 par une canalisation 207.

A la sortie de l'enceinte 202, on achemine:

– la première fraction contenant le sorbitol très pur par une canalisation 208 vers un réservoir de stockage non montré et

– la deuxième fraction contenant du sorbitol, des sucres non hydrogénés ainsi que des di- et polysaccharides hydrogénés par une canalisation 209 vers l'entrée de l'enceinte 203.

Les moyens propres à assurer la circulation des divers sirops à l'intérieur de l'installation ne sont pas montrés.

L'hydrolysat d'amidon entrant dans la constitution de la matière première présente, de préférence, une teneur en dextrose vrai comprise entre 65 et 97% et, plus préférentiellement encore, entre 70 et 95%. Des hydrolysats d'amidon particulièrement avantageux sont constitués par les hydrols de premier et de deuxième jet obtenus lors de la cristallisation du dextrose.

Dans ce qui précède et dans ce qui suit, les pourcentages indiqués s'entendent, sauf mention contraire, sur la matière sèche des sirops.

L'étape d'hydrogénation catalytique est réalisée de façon connue en soi, notamment sur des catalyseurs au ruthénium ou au nickel de Raney. De préférence, elle est effectuée sur un catalyseur au nickel de Raney, à une pression d'hydrogène comprise entre 40 et 70 kg/cm² et à une température d'environ 100 à 150 °C.

L'étape de séparation chromatographique peut s'effectuer, de manière connue en soi, de façon discontinue ou continue (lit mobile simulé), sur des adsorbants du type résines cationiques, fortement acides, chargés en ions alcalins ou alcalino-terreux ou du type zéolithes, chargées en ions ammonium, sodium, potassium calcium, strontium, baryum.

Des exemples de tels procédés de séparation chromatographique sont décrits dans les brevets US 3 044 904, US 3 416 961, US 3 692 582, FR 2 391 754, FR 2 099 336, US 2 985 589, US 4 024 331, US 4 226 977, US 4 293 346, US 4 157 267, US 4 182 633, US 4 332 633, US 4 405 445, US 4 412 866 et US 4 422 881.

Suivant un mode de réalisation préféré, l'étape de séparation chromatographique est effectuée par mise en œuvre du procédé et de l'appareillage décrits dans le brevet US N° 4 422 881 et son correspondant français N° 79 10 563 dont la Société Demanderesse est la titulaire.

Quel que soit le procédé de séparation chromatographique retenu, on a recours, de préférence, à titre d'adsorbant, à une résine cationique forte mise sous forme calcium et ayant un taux de divinylbenzène d'environ 4 à environ 10%.

De préférence, l'étape d'hydrolyse acide de la susdite deuxième fraction est effectuée sur un catalyseur acide fixe et elle est conduite de façon telle que plus de 40% en poids des disaccharides et polysaccharides hydrogénés présents soient hydrolysés.

On peut utiliser, pour ce faire, tout type d'enceinte réactionnelle et tout réactif ou catalyseur acide capable d'assurer l'hydrolyse des disaccharides et polysaccharides hydrogénés.

De préférence, ladite hydrolyse est effectuée en continue en faisant passer le sirop contenant les polyols à hydrolyser sur un lit fixe comprenant des catalyseurs acides du type alumino-silicates, silice, alumine ou résines cationiques, à une température et durant un temps suffisants pour atteindre le résultat recherché indiqué plus haut.

Avantageusement, l'hydrolyse est conduite sur des résines cationiques fortes sous forme acide, à une température comprise entre 50 et 120 °C, le débit de passage sur le lit catalytique acide étant réglé de façon à obtenir un taux d'hydrolyse des di- et polysaccharides hydrogénés au moins égal à 60% et, de préférence, au moins égal à 80%.

La teneur en matières sèches de la susdite deuxième fraction qui est soumise à l'étape d'hydrolyse et qui est recyclée est généralement comprise entre 1,5% et 30% et, de préférence, entre 2,5% et 20%.

Le procédé ainsi décrit permet d'obtenir des sirops de sorbitol présentant une richesse en sorbitol supérieure à 98%, et même supérieure à 99%, qui se caractérisent essentiellement par une teneur minime en sucres réducteurs. Ces sirops, qui présentent à la sortie de l'enceinte 202, une teneur en matières sèches comprise entre 20% et 50% et, de préférence, entre 25% et 40%, peuvent ensuite être évaporés jusqu'à la matière sèche de commercialisation ou être évaporés plus complètement en vue de la production de sorbitol cristallisé.

L'invention pourra être encore mieux comprise à l'aide des exemples qui suivent et des figures illustratives qui s'y rapportent, lesdits exemples étant relatifs à des modes de réalisation avantageux de l'invention.

Exemple 1

Préparation de sorbitol pur à partir d'un hydrolysat d'amidon préparé par hydrolyse enzymatique double à l'alphaamylase et l'amyloglucosidase et présentant une richesse en dextrose vrai égale à 94,5%, sa composition étant:

| | |
|---|---|
| Matière sèche (MS) : | 74,0% |
| Dextrose : | 94,5% |
| DP 2 : | 4,0 |
| DP 3 : | 1,0 |
| DP > 3 : | 0,5 |

étant entendu que «DP» signifie le degré de polymérisation d'un constituant donné de l'hydrolysat.

a) Etape d'hydrogénation

Le susdit hydrolysat, après purification sur résines échangeuses d'ions et sur charbon actif, est hydrogéné sur nickel de Raney, à une pression d'hydrogène de 45 kg/cm$^2$ et à une température de 125 °C.

Il présente, après purification conventionnelle, la composition suivante:

| | |
|---|---|
| DP > 2 : | 1,3 |
| DP 2 : | 5,6 |
| Glycérine : | 0,2 |
| Mannitol : | 1,0 |
| Autres hexitols : | 0,4 |
| Sorbitol : | 93,5 |
| Sucres réducteurs : | 0,08 |
| Sucres totaux* : | 2,74 |

*déterminés par la méthode analytique définie par le FOOD CHEMICAL CODEX, Seconde Edition, page 791.

b) Etape de séparation chromatographique

Le susdit hydrolysat d'amidon hydrogéné est ensuite envoyé sur une installation de séparation chromatographique continue dont les détails de la constitution et du fonctionnement sont ceux décrits dans le brevet US N° 4 422 881 et dans le brevet correspondant français est le N° 79 10563, ces détails n'étant repris isi que dans la mesure où la compréhension du présent texte l'exige.

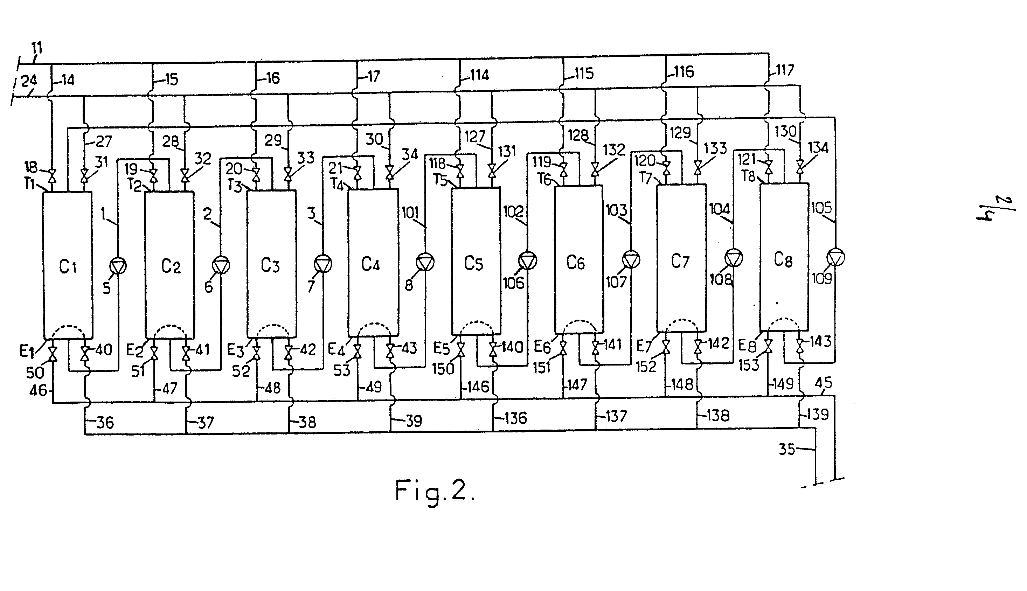

Elle comporte, comme montré à la figure 2 du brevet américain (reprise ici en tant que figure 2 pour l'explicitation détaillée de laquelle on se reporte au brevet américain), huit colonnes ou étages C$_1$ à C$_8$ de 200 litres chacune, garnies d'adsorbant du type résines cationiques fortes sous forme calcium et de fine granulométrie (0,2 à 0,4 microns).

De par le calage des électrovannes, on forme une zone I de désorption de deux étages, une zone II d'adsorption de quatre étages et une zone III d'enrichissement des polyholosides hydrogénés de deux étages.

Un dispositif de fermeture maintient dans la configuration adoptée une étanchéité totale entre la zone III, zone d'enrichissement à l'extrémité de laquelle sont récupérés les polyholosides hydrogénés et la zone I de désorption du sorbitol, zone en tête de laquelle est introduite l'eau de désorption.

Ce dispositif de fermeture assure le sens du passage de la phase liquide sur l'adsorbant sélectif et évite surtout la contamination du sorbitol pur par des traces de polyholosides, dont la vitesse de migration au sein de la résine est largement supérieure à celle des di- ou triholosides hydrogénés.

Une minuterie non montrée ajustée à 23 minutes 50 secondes assure pour les débits indiqués ci-après une alimentation en eau suffisante pour effectuer le désucrage du premier étage de la zone de désorption, une alimentation en un volume d'hydrolysat d'amidon hydrogéné ou HAH compatible avec le volume d'adsorbant et sa capacité d'adsorption, de façon à obtenir un taux d'extraction des polyholosides hydrogénés supérieur à 99% et un taux d'extraction du sorbitol au moins égal à 90% du sorbitol présent dans l'hydrolysat hydrogéné d'alimentation. Ces taux sont maintenus constants en ajustant le débit de la pompe d'extraction non montrée du sorbitol adsorbé. La sortie de la fraction polyholosides s'effectue à pression atmosphérique et son débit, constant, résulte de la différence entre les débits d'alimentation et le débit d'extraction.

L'hydrolysat d'amidon hydrogéné qui est introduit dans l'installation en tête de l'étage d'adsorption, présente une teneur en matières sèches égale à 64,5%. La température à l'intérieur des colonnes de séparation est maintenue à environ 90 °C.

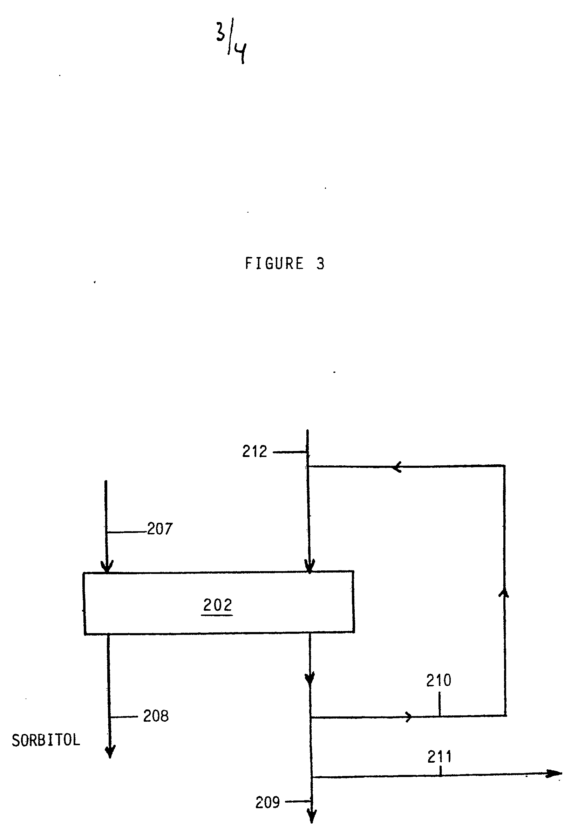

Sur le schéma synoptique de la figure 3 montre l'installation de la figure 2 schématiquement en 202 (les mêmes références désignant les mêmes éléments pour les parties communes que dans la figure 1). L'installation de chromatographie comporte, en plus des éléments constitutifs déjà montrés figure 1, une canalisation 210 de recyclage des excédents d'eau et une canalisation 211 par laquelle sont éliminées du circuit les fractions polyholosides de DP > 4 extraits à très faible MS.

L'alimentation de l'installation en eau s'effectue par une canalisation 212.

Les flèches portées sur les canalisations indiquent le sens de circulation.

L'ensemble fonctionne comme suit:
- le HAH destiné au fractionement chromatographique est acheminé par la canalisation 207 à un débit de 120 l/heure et présente une teneur en matières sèches de 64,5%,
- l'eau est amenée par la canalisation 212 avec un débit de 330 l/heure,
- le sorbitol pur est récupéré par la canalisation 208 avec un débit de 240 l/heure, sa teneur en matières sèches étant de 34,15%,
- la quantité totale de liquides extraits de l'installation 202 l'est avec un débit total de 386 l/heure, se composant successivement:

· d'une fraction d'excédent d'eau de 176 l/heure qui est recyclée par la canalisation 210 à la tête de l'installation,

· d'une fraction polyholosides éliminée par la canalisation 211, dont le débit est de 114 l/heure et la M.S. d'environ 1%,

· d'une fraction polyholosides acheminée par la canalisation 209 vers l'installation d'hydrolyse avec un débit de 96 l/heure, la teneur en M.S. étant de 3,5%.

Les analyses des sorties de sorbitol et de polyholosides hydrogénés sont présentées dans les tableaux I et II.

Tableau I
Analyse de la sortie sorbitol

| Temps (minutes) | Brix | DP 2 % | Glycérine % | Mannitol % | Sorbitol % |
|---|---|---|---|---|---|
| 0 | 56,5 | 0,4 | 0,3 | 1,0 | 98,3 |
| 3 | 47,5 | 0,3 | 0,3 | 0,9 | 98,5 |
| 6 | 41,5 | 0,3 | 0,3 | 0,8 | 98,6 |
| 12 | 27,0 | 0,2 | 0,2 | 0,5 | 99,1 |
| 15 | 21,5 | 0,2 | 0,1 | 0,5 | 99,2 |
| 18 | 16,0 | – | 0,1 | 0,4 | 99,5 |
| 21 | 12,5 | – | 0,1 | 0,3 | 99,6 |
| 23'50'' | 10,0 | – | – | 0,3 | 99,7 |

De l'examen du tableau I, il apparaît que le sorbitol a été extrait pendant 23 minutes 50 secondes avec une pureté moyenne supérieure à 98% et une teneur en matières sèches moyenne de 34,15%.

La valeur du «brix» donne le pourcentage approximatif de matière sèche; elle est déterminée par l'indice de réfraction.

Tableau II
Analyse de la sortie polyholosides

| Temps (minutes) | Brix | Pouvoir rotatoire (α) D | DP > 3 % | DP 3 % | DP 2 % | Sorbitol % |
|---|---|---|---|---|---|---|
| 0 | – | | | | | |
| 2 | – | | | | | |
| 4 | – | | | | | |
| 6 | – | | | | | |
| 8 | – | | | | | |
| 10 | – | | | | | |
| 12 | – | +0,166 | +++ | ++ | + | |
| 14 | 0,7 | +0,629 | +++ | ++ | + | |
| 16 | 1,5 | +1,038 | 35,9 | 30,9 | 22,6 | 10,6 |
| 18 | 2 | +1,079 | 24,0 | 29,5 | 33,7 | 12,8 |
| 20 | 2,5 | +2,209 | 21,0 | 26,0 | 43,7 | 9,3 |
| 22 | 3,4 | +2,807 | 16,1 | 21,2 | 53,4 | 9,3 |
| 23'50'' | 4,0 | +2,604 | 13,5 | 19,0 | 57,0 | 10,5 |

Le pouvoir rotatoire spécifique (α) D est déduit de l'angle de rotation lu sur un polarimètre.

L'analyse détaillée des résultats réunis au tableau II et des indications fournies plus haut en rapport avec la figure 3, à propos des polyholosides hydrogénés, montre par ailleurs que:

– pendant les onze premières minutes, soit un équivalent de 176 l/h, la sortie polyholosides a pu être recyclée pour le désucrage de l'étage suivant,

– de la minute 11 à la minute 18, la fraction extraite, contenant la majeure partie des polyholosides hydrogénés de DP supérieur ou égal à 4 a été éliminée du système, soit 114 l/h à 1% de matières sèches,

– puis, de la minute 18 à l'instant correspondant à 23 minutes 50 secondes, la fraction polyols a été récupérée pour l'étape d'hydrolyse, soit 96 litres à une matière sèche de 3,6%.

Le bilan global, qui apparaît à l'examen du schéma de la figure 3 et des tableaux I et II, se résume comme indiqué ci-après.

Le système de séparation chromatographique a été alimenté comme suit:

|  | Hydrolysat d'amidon hydrogéné | Eau |
|---|---|---|
| Débit | 120 l/h | 330 l/h |
| Densité | 1,269 | |
| % Matières sèches | 64,5 | |
| Débit massique | 98,22 kg/h | |

Il a été retiré de ce système:

|  | Sorbitol | Polyols supérieurs rejetés | Polyols récupérés |
|---|---|---|---|
| Débit | 240 l/h | 114 l/h | 96 l/h |
| Densité | 1,138 | 1,0 | 1,02 |
| % M.S. | 34,15 | 1,0 | 3,6 |
| Débit massique | 93,27 kg/h | 1,14 kg/h | 3,53 kg/h |

Ces résultats correspondent à un taux d'extraction de sorbitol présent dans l'hydrolysat d'amidon hydrogéné supérieur à 99%.

c) Etape d'hydrolyse continue des polyols récupérés

La fraction de polyols récupérés est ensuite acheminée par la canalisation 209 dans une enceinte d'hydrolyse 203, constituée par une colonne identique à celles équipant l'installation de séparation chromatographique illustrée par la figure 2.

Cette colonne est garnie d'une résine cationique de très fine granulométrie (0,1–0,2 microns) mise sous forme $H^+$.

Le débit d'alimentation est réglé à environ 90 litres/heure.

L'enceinte d'hydrolyse est maintenue à une température d'environ 110 °C.

Dans ces conditions, le sirop hydrolysé sort de l'enceinte 203 avec un taux de sucres réducteurs égal à 17,0 grammes/litre d'équivalent glucose, ce qui équivaut à un taux d'hydrolyse de 91,4%, puisqu'un test de dosage des sucres réducteurs effectué après hydrolyse complète (effectuée après deux heures à reflux en présence de $SO_4H_2$ normal) révèle un équivalent glucose de 18,6 grammes par litre.

Les analyses chromatographiques du sirop à l'entrée et à la sortie de l'enceinte d'hydrolyse 203 donnent les résultats indiqués ci-après.

La composition du sirop acheminé par la canalisation 209 vers l'enceinte 203 est la suivante:

| Sorbitol | 9 |
|---|---|
| Mannitol | 0,8 |
| DP 2 | 55 |
| DP 3 | 15,2 |
| DP > 3 | 20 |

et celle du sirop sortant de l'enceinte 203 par la canalisation 204b. déterminée sur trois prélèvements, la suivante:

| Sorbitol | 41 | 42 | 39,6 |
|---|---|---|---|
| Mannitol | 2,3 | 2,5 | 2,8 |
| DP 2 | 5 | 7 | 5 |
| DP 3 | 5 | 6,5 | 7,0 |
| Glucose | 46,4 | 52 | 45,6 |

d) Recyclage par la canalisation 204b du sirop ainsi hydrolysé vers l'enceinte d'hydrogénation 201, ce qui revient à une dilution de l'hydrolysat d'amidon de départ.

Dans ce cas, on achemine par la canalisation 204 une matière première constituée d'hydrolysat d'amidon non encore traité et de sirop hydrolysé sortant de l'enceinte 203.

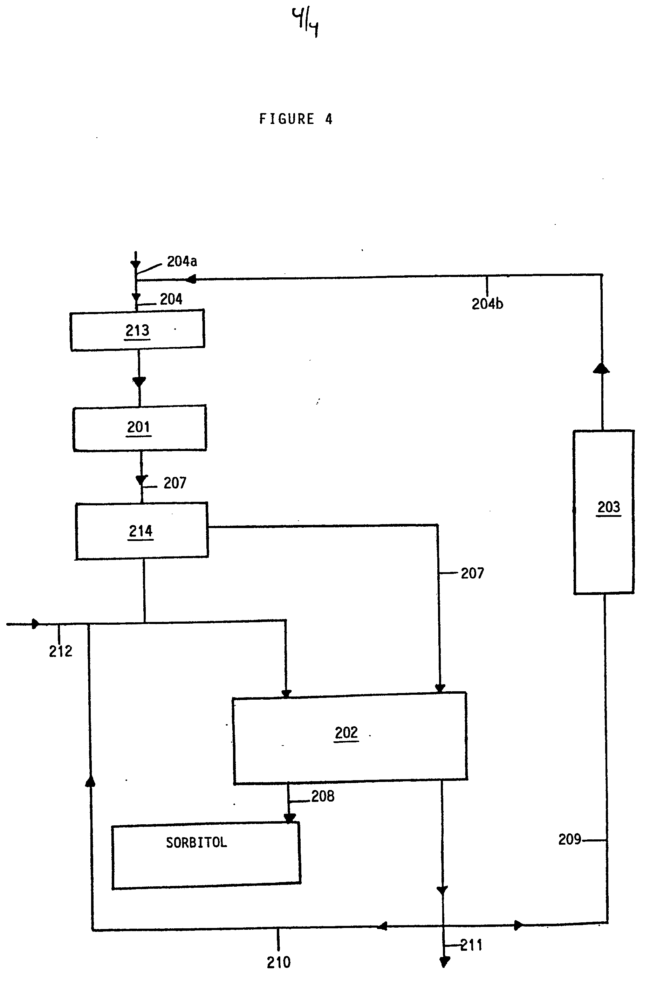

A la figure 4, on a représenté schématiquement les éléments principaux de l'installation correspondante, les mêmes éléments étant désignés par les mêmes chiffres de référence que dans les figures 1 et 3.

Deux stades de purification 213 et de purification évaporation 214 sont représentés sur respectivement les canalisations 205 et 207.

Pour amener la matière première introduite sur l'enceinte 201 à une teneur en matières sèches d'environ 43%, ce qui correspond aux conditions optimales de la réaction d'hydrogénation, on achemine par la canalisation 204a, à un débit de 140,4 kg/h, l'hydrolysat d'amidon non encore traité et présentant une teneur en matières sèches de 70%, ce qui, après réunion avec le débit de 97,9 kg/h de sirop sortant de l'enceinte 203 avec une teneur en matières sèches de 3,6%, fournit une matière première d'une teneur en matières sèches de 42,7% avec un débit de 238,3 kg/h.

Après purification et évaporation à l'étape 214, on achemine ainsi vers l'installation 202, par la canalisation 207, un sirop hydrogéné d'une teneur en matières sèches de 64,5% à un débit de 120 l/heure.

Comme déjà indiqué en rapport avec la figure 3:
— l'installation 202 est alimentée avec 330 l/heure d'eau arrivant par la canalisation 212 auxquels s'ajoutent les 176 l/heure d'eau excédentaire recyclée par la canalisation 210, soit un total de 506 l/heure;
— on extrait de l'installation 202:
  · 114 l/heure d'une fraction de polyols supérieurs éliminée du système par la canalisation 211 et présentant une teneur en matières sèches d'environ 1%.
  · 96 l/heure à une teneur en matières sèches de 3,6% de polyols amenés vers l'enceinte d'hydrolyse 203 par la canalisation 209.
L'ensemble du dispositif ainsi décrit a été mis en fonctionnement continu.
Dans les tableaux III, IV et V ci-après, on a réuni les valeurs enregistrées respectivement en rapport avec les analyses de l'hydrolysat d'amidon hydrogéné avant séparation chromatographique avec les analyses de la fraction sorbitol récupérée et avec les analyses de la fraction devant être soumise à l'hydrolyse, au cours de cinq prélèvements successifs différents.

Tableau III
Hydrolysat hydrogéné avant chromatographie

| DP > 2 | 1,6 | 1,6 | 1,65 | 1,5 | 1,7 |
|---|---|---|---|---|---|
| DP 2 | 3,5 | 3,5 | 2,85 | 3,4 | 3,6 |
| Glycérine | 0,1 | 0,1 | 0,2 | 0,2 | 0,2 |
| Mannitol | 0,7 | 0,9 | 1,0 | 0,8 | 1,0 |
| Autres hexitols | 0,3 | 0,3 | 0,4 | 0,2 | 0,4 |
| SORBITOL | 93,8 | 93,6 | 93,9 | 93,9 | 93,1 |
| Sucres réducteurs | 0,09 | 0,07 | 0,065 | 0,06 | 0,092 |
| Sucres totaux | 1,5 | 1,45 | 1,50 | 1,33 | 1,33 |

Tableau IV
Analyse de la fraction sorbitol

| DP > 2 | — | — | — | — | — |
|---|---|---|---|---|---|
| DP 2 | 0,2 | 0,3 | 0,3 | 0,25 | 0,3 |
| SORBITOL | 98,6 | 98,2 | 98,4 | 98,45 | 98,1 |
| Mannitol | 0,7 | 0,9 | 0,9 | 0,8 | 1,0 |
| Glycérine | 0,2 | 0,2 | 0,2 | 0,2 | 0,2 |
| Autres hexitols | 0,3 | 0,4 | 0,3 | 0,3 | 0,4 |
| Sucres réducteurs | 0,017 | 0,028 | 0,010 | 0,020 | 0,012 |
| Sucres totaux | 0,11 | 0,09 | 0,14 | 0,12 | 0,17 |

Tableau V
Analyse de la fraction soumise à l'hydrolyse

| DP > 3 | 20,2 | 20,2 | 16,9 | 16,4 |
|---|---|---|---|---|
| DP 3 | 18,4 | 16,1 | 14,1 | 13,8 |
| DP 2 | 43,2 | 49,2 | 55,8 | 55,5 |
| SORBITOL | 17,6 | 13,5 | 12,1 | 13,3 |
| Mannitol | 0,6 | 1,0 | 1,1 | 1,0 |
| Autres hexitols | — | — | — | — |
| Sucres réducteurs | 0,83 | 0,30 | 0,25 | 0,35 |
| Sucres totaux | 26,4 | 28,0 | 33,5 | 30,5 |

La fraction sorbitol ainsi obtenue a été comparée à cinq qualités de sorbitol liquide obtenues par hydrogénation de dextrose selon l'art antérieur et dont les analyses sont présentées dans le tableau VI ci-après.

Tableau VI
Analyses de sirops de sorbitol obtenues par hydrogénation de dextrose selon l'art antérieur

| DP > 2 | 0,06 | 0,15 | 0,40 | 0,25 | 0,30 |
|---|---|---|---|---|---|
| DP 2 | 0,40 | 0,40 | 0,40 | 0,6 | 0,50 |
| SORBITOL | 97,84 | 98,8 | 97,55 | 97,35 | 97,40 |
| Autres hexitols | 0,40 | 0,50 | 0,15 | 0,2 | 0,20 |
| Mannitol | 1,0 | 0,80 | 1,1 | 1,1 | 1,1 |
| Glycérine | 0,30 | 0,03 | 0,4 | 0,5 | 0,5 |
| Sucres réducteurs | 0,058 | 0,040 | 0,052 | 0,047 | 0,050 |
| Sucres totaux | 0,26 | 0,26 | 0,22 | 0,26 | 0,30 |

On peut constater, en comparant les tableaux IV et VI que la qualité de sorbitol obtenue par le procédé selon l'invention:
- a une pureté moyenne en sorbitol supérieure,
- présente une teneur en produits de DP égal ou supérieur à 2 notablement plus basse,
- et présente une teneur en sucres réducteurs nettement moins importante (0,017 en moyenne contre 0,050).

Un taux de sucres réducteurs, aussi faible dans le cas du procédé selon l'invention, ne pourrait être obtenu que très difficilement par hydrogénation directe. Ce taux laisserait en effet supposer un degré d'hydrogénation de 99,98%, ce qui ne peut être atteint qu'avec des durées d'hydrogénation fortement prolongées et, par conséquent, non économiques.

Exemple 2

Cet exemple décrit la production de sorbitol pur à partir d'un hydrol de premier jet de cristallisation du dextrose, présentant une teneur en dextrose vrai égale à 86,7%.

a) Etape d'hydrogénation catalytique de l'hydrol

L'hydrol, obtenu après cristallisation d'un premier jet de dextrose monohydrate, présente une teneur en matières sèches de 74% et la composition suivante:

| | |
|---|---|
| DP > 3 | 2,7 |
| DP 3 | 1,8 |
| DP 2 | 8,5 |
| Dextrose | 86,70 |
| Fructose | 0,3 |

Après purification et hydrogénation, la composition du sirop devient:

| | |
|---|---|
| DP > 2 | 4,6 |
| DP 2 | 7,5 |
| Glycérine | — |
| Mannitol | 1,0 |
| Autres hexitols | 0,2 |
| Sorbitol | 86,7. |

b) Etape de séparation chromatographique liquide continue

Cette séparation est effectuée sur le dispositif et suivant le procédé décrit à l'exemple 1 en rapport avec la figure 3.

L'installation de chromatographie est alimentée:
- en hydrol hydrogéné par la canalisation 207 à un débit de 120 l/heure, l'hydrol présentant une teneur en matières sèches de 62% et une densité d = 1,267, soit 94,26 kg/heure,
- en eau par la canalisation 212 à un débit de 330 l/heure,
- en fraction excédent d'eau recyclée par la canalisation 210 à un débit de 176 l/heure.

Il est retiré de cette installation:
- du sorbitol pur par la canalisation 208 à un débit de 220 l/heure, la teneur en matières sèches étant de 32% et la densité de 1,137, soit 80 kg/heure.
- une fraction de polyols rejetée par la canalisation 211 à un débit de 100 l/heure, la teneur en matières sèches étant de 1,5% et la densité de 1,00, soit 1,5 kg/heure,
- une fraction de polyols soumis à hydrolyse par la canalisation 209 à un débit de 130 l/heure, avec une teneur en matières sèches de 8,8% et une densité de 1,02, soit 11,7 kg/heure.

L'ensemble donne une extraction calculée sur le sorbitol de 96,2%.

c) Etape d'hydrolyse continue

Les courants de polyols récupérés sont acheminés par la canalisation 209 à l'enceinte d'hydrolyse 203 telle que décrite à l'exemple 1. La température y est maintenue à 115 °C. Le degré d'hydrolyse des polyholosides mesuré par les sucres réducteurs en comparaison avec le produit hydrolysé à reflux deux heures en présence d'acide sulfurique normal, a toujours été supérieur à 90%.

d) Etape de dilution, au moyen du sirop sortant de l'enceinte 203 et acheminé par la canalisation 204b, de l'hydrol amené par la canalisation 204a avant purification.

Dans le circuit intégré tel que présenté dans l'exemple 1, la fraction polyols hydrolysée est utilisée pour la dilution de l'hydrol avant purification, l'ensemble constituant la matière première soumise à hydrogénation.

Du point de vue pratique, 112 kg/h d'hydrol à 74% de matières sèches sont ainsi dilués avec 130 l/h de polyols hydrolysés. La solution ainsi diluée titre 38,7% de matières sèches.

Après hydrogénation, purification et évaporation à 62% de matières sèches, le sirop est envoyé vers l'installation de chromatographie continue.

Après plusieurs jours de fonctionnement, on a obtenu les résultats réunis dans les tableaux VII (pour trois prélèvements), VIII (pour cinq prélèvements), et IX (pour cinq prélèvements).

Tableau VII
Analyse de la matière première constituée par l'hydrol dilué par les polyholosides hydrogènes
Hydrolyses puis hydrogène

| Nature du constituant | Teneur en % dans le sirop | | |
|---|---|---|---|
| DP > 2 | 4,6 | 2,9 | 5,6 |
| DP 2 | 7,1 | 7,5 | 7,7 |
| Glycérine | 0,1 | 0,2 | 0,1 |
| Mannitol | 1,1 | 0,9 | 1,0 |

Tableau VII (suite)

| Nature du constituant | Teneur en % dans le sirop | | |
|---|---|---|---|
| Autres hexitols | 0,3 | 0,2 | 0,3 |
| SORBITOL | 86,8 | 88,3 | 85,3 |
| Sucres réducteurs | 0,08 | 0,06 | 0,07 |
| Sucres totaux | 4,36 | 4,7 | 5,5 |

Tableau VIII
Analyse du sorbitol obtenu

| Nature du constituant | Teneur en % dans le sorbitol | | | | |
|---|---|---|---|---|---|
| DP 3 | — | — | — | — | — |
| DP 2 | 0,4 | 0,3 | 0,6 | 0,2 | 0,4 |
| Mannitol | 1,0 | 1,1 | 0,9 | 0,7 | 1,1 |
| Autres hexitols | 0,3 | 0,2 | 0,2 | 0,3 | 0,1 |
| Glycérine | 0,15 | 0,1 | 0,1 | 0,2 | 0,1 |
| SORBITOL | 98,15 | 98,3 | 98,2 | 98,6 | 98,6 |
| Sucres réducteurs | 0,025 | 0,010 | 0,022 | 0,010 | 0,040 |
| Sucres totaux | 0,11 | 0,15 | 0,20 | 0,09 | 0,13 |

Tableau IX
Analyse des polyols acheminés avant hydrolyse

| Nature du constituant | Teneur en % | | | | |
|---|---|---|---|---|---|
| DP > 3 | 18,7 | 20,2 | 21,3 | 16,9 | 16,4 |
| DP 3 | 15,9 | 16,1 | 17,0 | 14,2 | 13,7 |
| DP 2 | 48,0 | 49,2 | 51,9 | 55,8 | 57,4 |
| SORBITOL | 17,4 | 14,5 | 9,8 | 13,1 | 13,5 |
| Sucres réducteurs | 0,17 | 0,14 | 0,11 | 0,14 | 0,14 |
| Sucres totaux | 27,1 | 33,8 | 34,5 | 29,6 | 28,7 |

La composition plus détaillée d'un échantillon de cette fraction de polyols avant hydrolyse est donnée dans le tableau X:

Tableau X

| Nature du constituant | Teneur en % |
|---|---|
| Sorbitol | 12,8 |
| Mannitol | 1,9 |
| DP 2 | 50,4 |
| DP 3 | 14,9 |
| DP 4 | 3,0 |
| DP 5 | 2,1 |
| DP 6 | 2,9 |
| DP 7 | 2,8 |
| DP 9 | 1,1 |
| DP 10 à DP 20 | 5,6 |
| DP > 20 | 0,9 |

De toutes ces analyses, il ressort à nouveau que la qualité de sorbitol produite dans ces conditions avec un rendement total à partir d'un hydrol de première cristallisation de dextrose est au moins égale à celle obtenue par hydrogénation d'un dextrose pur.

On se reportera, à titre de comparaison, aux compositions, données dans l'exemple 1, de sorbitol obtenu par hydrogénation de dextrose.

Les polyols extraits pendant tout le fonctionnement équilibré de la chromatographie avaient une teneur en sorbitol vrai inférieure à 15%, ce qui indique une extraction efficace du sorbitol, supérieure à 90% de la teneur en sorbitol de la matière travaillée.

Exemple 3

Cet exemple illustre la production de sorbitol pur à partir d'un hydrol H2 de deuxième jet de cristallisation du dextrose, titrant environ 78% de dextrose vrai.

On se reporte toujours aux figures 1 et 3.

a) Etape d'hydrogénation

Le sirop obtenu après purification et hydrogénation présente la composition indiquée au tableau XI:

Tableau XI

| Nature du constituant | Teneur en % |
|---|---|
| DP > 3 | 3,5 |
| DP 3 | 3,1 |
| DP 2 | 11,1 |
| Mannitol | 1,0 |
| Autres hexitols | 0,3 |
| Sorbitol | 78,0 |
| Sucres réducteurs | 0,48 |
| Sucres totaux | 7,1 |

b) Etape de chromatographie

Le susdit sirop est alimenté à un débit de 103,2 l/h pour une teneur en matières sèches de 63,5% par la canalisation 207 vers l'installation de chromatographie liquide continue 202 décrite précédemment; la densité du sirop est de 1,264.

Le débit d'alimentation en eau par la canalisation 212 de cette installation est de 341 l/h, auquel on doit ajouter un débit de 166 l/h d'eau de recyclage (excédent d'eau provenant du désucrage total lors de la séquence précédente du premier étage de désorption de sorbitol pur) amenée par la canalisation 210.

La pompe d'extraction de sorbitol est réglée à 200 l/h pour obtenir à la fois une excellente qualité de sorbitol ainsi qu'un taux d'extraction maximum.

On extrait donc 200 l/h de sorbitol d'une teneur en matières sèches de 28,2%, soit 62,04 kg/heure par la canalisation 208.

On extrait par la canalisation 209 un sirop devant être soumis à hydrolyse, le débit étant de 134 l/heure, la densité de 1,04 et la teneur en matières sèches de 13,7%, soit 18,9 kg/heure.

On extrait par la canalisation 211 un sirop rejeté avec un débit de 160 l/heure, la teneur en matières sèches étant de 1,7%, soit 1,87 kg/heure.

On a analyse en fonction du temps tant le sorbitol extrait de l'installation que la sortie de la fraction polyols.

Les résultats sont réunis dans les tableaux XII et XIII.

Tableau XII
Analyse du sorbitol

| Temps | Brix | DP > 3 | DP 2 | Mannitol | Autres hexitols | Sorbitol |
|---|---|---|---|---|---|---|
| 0 | 56 | 0,2 | 0,5 | 1,1 | 0,3 | 97,9 |
| 2 | 54 | 0,2 | 0,4 | 1,0 | 0,3 | 98,0 |
| 4 | 48 | 0,2 | 0,4 | 1,0 | 0,4 | 97,9 |
| 6 | 4,0 | 0,1 | 0,3 | 1,0 | 0,3 | 98,1 |
| 8 | 35,5 | 0,1 | 0,2 | 1,0 | 0,3 | 98,4 |
| 10 | 31 | | 0,2 | 1,0 | 0,3 | 98,5 |
| 12 | 26 | | 0,2 | 0,9 | 0,4 | 98,5 |
| 14 | 21 | | 0,3 | 0,9 | 0,3 | 98,4 |
| 16 | 16 | | 0,3 | 0,8 | 0,3 | 98,6 |
| 20 | 10 | | 0,2 | 0,7 | 0,3 | 98,9 |
| 22 | 8 | | 0,2 | 0,6 | 0,3 | 98,9 |
| 24 | 6 | | 0,2 | 0,6 | 0,3 | 98,9 |

Tableau XIII
Analyse de la fraction polyols

| Temps | Brix | DP > 3 | DP 3 | DP 2 | Mannitol | Autres hexitols | Sorbitol |
|---|---|---|---|---|---|---|---|
| 2 | | | | | | | |
| 4 | | | | | | | |
| 6 | | | | | | | |
| 8 | | | | | | | |
| 10 | | | | | | | |
| 12 | | | | | | | |
| 14 | 2 | | | | | | |
| 16 | 5 | | | | | | |
| 18 | 7,5 | | | | | | |
| 20 | 10,5 | | | | | | |
| 22 | 21,0 | 20 | 15,7 | 5,2 | 1,3 | 0,4 | 10 |
| 24 | 28,0 | 21 | 16,7 | 54,6 | 1,4 | 0,4 | 6 |

c) Etape d'hydrolyse continue

Le sirop récupéré aux fins d'hydrolyse à la sortie de l'installation 202 est acheminé vers l'enceinte d'hydrolyse tel que décrit dans l'exemple 1.

La température y est maintenue à 110 °C. Le degré d'hydrolyse des polyholosides pour un débit de 130 l/h sur un lit de 20 litres d'adsorbant à toujours été supérieur à 90%.

Le degré d'hydrolyse est, comme dans les exemples précédentes, obtenu en comparant le taux de sucres réducteurs exprimé en équivalent glucose, trouvé sur le sirop sortant de l'enceinte d'hydrolyse, avec celui trouvé sur un échantillon soumis à l'action d'acide sulfurique normal sous reflux.

d) Etape de dilution de l'hydrol H2 amené par la canalisation 204a, avant hydrogénation avec le sirop sortant de l'enceinte d'hydrolyse et amené par la canalisation 204b.

On dilue 93,4 kg/heure d'hydrol H2 à 74% de matières sèches avec les 134 l/heure de polyols hydrolysés à 13,7% de matières sèches, amenés par la canalisation 204b, la solution obtenue représentant 230 kg/heure d'un sirop à 38% de matières sèches environ.

Ce sirop est hydrogéné, purifié et évaporé à 63,5% de matières sèches, puis acheminé vers l'installation de chromatographie continue 202.

Après plusieurs jours de fonctionnement, on a effectué l'analyse de cinq prélèvements de la matière première constituée par l'hydrol dilué par le sirop amené par 204b (Tableau XIV) et hydrogéné, de cinq prélèvements du sorbitol obtenu (Tableau XV) et de quatre prélèvements du sirop amené par la canalisation 209 (Tableau XVI).

Tableau XIV
Hydrol dilué par les polyholosides hydrogènes hydrolysés puis hydrogène

| Nature du constituant | Teneur en % | | | | |
|---|---|---|---|---|---|
| DP > 3 | 5,2 | 6,6 | 4,5 | 5,4 | 4,3 |
| DP 3 | 3,3 | 4,3 | 3,1 | 2,8 | 3,2 |
| DP 2 | 10,2 | 14,0 | 11,7 | 12,4 | 11,7 |
| Mannitol | 1,2 | 1,3 | 1,2 | 1,3 | 1,5 |
| SORBITOL | 79,8 | 75,4 | 79,2 | 79,0 | 79,0 |
| Autres hexitols | 0,3 | 0,4 | 0,3 | 0,2 | 0,3 |
| Sucres réducteurs | 0,05 | 0,045 | 0,048 | 0,035 | 0,07 |
| Sucres totaux | 6,9 | 7,2 | 7,5 | 7,0 | 8,0 |

Tableau XV
Sorbitol

| Nature du constituant | Teneur en % | | | | |
|---|---|---|---|---|---|
| DP 3 | — | 0,1 | 0,05 | 0,05 | 0,1 |
| DP 2 | 0,2 | 0,2 | 0,2 | 0,3 | 0,3 |
| Mannitol | 0,9 | 0,9 | 1,1 | 1,1 | 1,2 |
| Autres hexitols | 0,5 | 0,2 | 0,2 | 0,3 | 0,1 |
| SORBITOL | 98,4 | 98,6 | 98,45 | 98,25 | 98,3 |
| Sucres réducteurs | 0,011 | 0,027 | 0,028 | 0,01 | 0,03 |
| Sucres totaux | 0,04 | 0,2 | 0,12 | 0,15 | 0,13 |

Des résultats d'analyses réunis aux tableaux XIV et XV, il ressort que la qualité de sorbitol produite dans ces conditions à partir d'hydrol H2 a une pureté au moins égale à celle obtenue par hydrogénation d'un dextrose pur et se caractérise par une teneur très faible en sucres réducteurs.

Tableau XVI
Sirop de polyols acheminé par la canalisation 209

| | | | | |
|---|---|---|---|---|
| DP > 3 | 19,2 | 21,7 | 21,0 | 20,7 |
| DP 3 | 15,9 | 16,9 | 16,4 | 16,4 |
| DP 2 | 51,6 | 56,1 | 54,6 | 54,8 |
| Mannitol | 3 | 2 | 1,8 | 2 |
| SORBITOL | 10 | 2,9 | 5,8 | 5,7 |
| Sucres réducteurs | 0,3 | 0,4 | 0,40 | 0,4 |
| Sucres totaux | 35,0 | 38,2 | 36,4 | 32,2 |

Des résultats réunis dans le tableau XVI, on déduit que les polyols extraits pendant tout le fonctionnement équilibré de l'installation de chromatographie avaient une teneur en sorbitol inférieure

à 10%, ce qui indique une extraction très efficace du sorbitol, supérieure à environ 94% de la teneur en sorbitol de la matière première.

Comme il va de soi et comme il résulte d'ailleurs déjà de ce qui précède, l'invention ne se limite nullement aux modes de réalisation et d'application qui ont été plus particulièrement envisagés; elle en embrasse, au contraire, toutes les variantes.

## Revendications

1. Procédé de préparation d'un sirop de sorbitol de très haute pureté, caractérisé par le fait qu'une matière première dont une première partie est constituée par un hydrolysat d'amidon, est soumise à un procédé comprenant en combinaison

    — une étape d'hydrogénation catalytique de la matière première,

    — une étape de séparation chromatographique de la matière première ayant subi l'hydrogénation en une première fraction contenant du sorbitol très pur et qui est recupérée et en une deuxième fraction contenant, outre du sorbitol, des sucres non hydrogénés ainsi que des di- et polysaccharides hydrogénés,

    — une étape d'hydrolyse acide de la susdite deuxième fraction, ce qui fournit un hydrolysat d'amidon partiellement hydrogéné qui est recyclé et qui constitue une deuxième partie de la matière première soumise à l'étape d'hydrogénation.

2. Procédé selon la revendication 1, caractérisé par le fait que l'hydrolysat d'amidon entrant dans la constitution de la matière première présente, de préférence, une teneur en dextrose vrai comprise entre 65 et 97% et, plus préférentiellement encore, entre 70 et 95%, des hydrolysats d'amidon particulièrement avantageux étant constitués par les hydrols de premier et de deuxième jet obtenus lors de la cristallisation du dextrose.

3. Procédé selon l'une des revendications 1 et 2, caractérisé par le fait que l'étape d'hydrogénation catalytique est réalisée sur des catalyseurs au ruthénium ou au nickel de Raney, de préférence sur un catalyseur au nickel de Raney, à une pression d'hydrogène comprise entre 40 et 70 kg/cm² et à une température d'environ 100 à 150 °C.

4. Procédé selon l'une des revendications 1 à 3, caractérisé par le fait que l'étape de séparation chromatographique s'effectue de façon discontinue ou continue sur des adsorbants du type résines cationiques, fortement acides, chargés en ions alcalins ou alcalino-terreux ou du type zéolithes, chargées en ions ammonium, sodium, potassium, calcium, strontium, baryum.

5. Procédé selon l'une des revendications 1 à 4, caractérisé par le fait que l'étape de séparation chromatographique met en œuvre plusieurs colonnes de chromatographie garnies d'adsorbant du type résines cationiques fortes sous forme calcium et de fine granulométrie, une zone I de désorption, une zone II d'adsorption et une zone III d'enrichissement des polyholosides hydrogénés étant formées par le calage des électrovannes situées sur les canalisations reliant la sortie d'une quelconque colonne à l'entrée de la colonne suivante, un dispositif de fermeture maintenant une étanchéité totale entre la zone III à l'extrémité de laquelle sont récupérés les polyholosides hydrogénés et la zone I de désorption du sorbitol, zone en tête de laquelle est introduite l'eau de désorption ledit dispositif de fermeture assurant le sens du passage de la phase liquide sur l'adsorbant sélectif et évitant surtout la contamination du sorbitol pur par des traces de polyholosides.

6. Procédé selon l'une des revendications 1 à 4, caractérisé par le fait que l'étape d'hydrolyse acide de la deuxième fraction est effectuée sur un catalyseur acide fixe et elle est conduite de façon telle que plus de 40% en poids des disaccharides et polysaccharides hydrogénés présents soient hydrolysés, ladite hydrolyse étant effectuée de préférence en continu en faisant passer le sirop contenant les polyols à hydrolyser sur un lit fixe comprenant des catalyseurs acides du type alumino-silicates, silice, alumine ou résines cationiques.

7. Procédé selon la revendication 6, caractérisé par le fait que l'hydrolyse est conduite sur des résines cationiques fortes sous forme acide, à une température comprise entre 50 et 120 °C, le débit de passage sur le lit catalytique acide étant réglé de façon à obtenir un taux d'hydrolyse des di- et polysaccharides hydrogénés au moins égal à 60% et, de préférence, au moins égal à 80%.

8. Procédé selon l'une des revendications 1 à 7, caractérisé par le fait que la teneur en matières sèches de la deuxième fraction qui est soumise à l'étape d'hydrolyse et qui est recyclée est généralement comprise entre 1,5% et 30% et, de préférence, entre 2,5% et 20%.

9. Installation pour la mise en œuvre du procédé selon l'une des revendications 1 à 8, caractérisée par le fait qu'elle comprend:

    — une enceinte 201 à l'intérieur de laquelle est réalisée l'étape d'hydrogénation catalytique,

    — une installation de séparation chromatographique 202 et

    — une enceinte réactionnelle 203 à l'intérieur de laquelle est réalisée l'étape d'hydrolyse.

## Patentansprüche:

1. Verfahren zur Herstellung von hochreinem Sorbitsirup, dadurch gekennzeichnet, dass ein Ausgangsmaterial, von dem ein erster Teil aus einem Stärkehydrolysat besteht, einem Verfahren unterworfen wird, welches eine Kombination der folgenden Stufen umfasst:

    — eine Stufe des katalytischen Hydrierens des Ausgangsmaterials,

    — eine Stufe der chromatographischen Auftrennung des hydrierten Ausgangsmaterials in eine erste Fraktion, die sehr reinen Sorbit enthält und welche gewonnen wird, und in eine zweite Fraktion, welche neben Sorbit nicht-hydrierte Zucker sowie hydrierte Di- und Polysaccharide enthält,

    — eine Stufe der sauren Hydrolyse der obgenannten zweiten Fraktion, welche zu einem teilweise hydrierten Stärkehydrolysat führt, welches im Kreislauf in das Verfahren rückgeführt wird und welches einen zweiten Teil des der Hydrierungsstufe unterworfenen Ausgangsmaterials darstellt.

2. Verfahren nach Anspruch 1, dadurch gekennzeichnet, dass das einen Bestandteil des Ausgangsmaterials darstellende Stärkehydrolysat vorzugsweise einen Gehalt an wahrer Dextrose aufweist, der zwischen 65 und 97%, und in noch höherem Masse bevorzugt zwischen 70 und 95%, liegt, wobei besonders vorteilhafte Stärkehydrolysate aus den Mutterlaugen oder Hydrolen bestehen, die bei der Kristallisation von Dextrose im zweiten oder dritten Kristallisationsgang erhalten worden sind.

3. Verfahren nach Anspruch 1 oder 2, dadurch gekennzeichnet, dass die Stufe der katalytischen Hydrierung über Ruthenium- oder Raney-Nickel-Katalysatoren, vorzugsweise über einem Raney-Nickel-Katalysator, bei einem zwischen 40 und 70 kg/cm² liegenden Wasserstoffdruck und bei einer Temperatur von etwa 100 bis 150 °C durchgeführt wird.

4. Verfahren nach einem der Ansprüche 1 bis 3, dadurch gekennzeichnet, dass die Stufe der chromatographischen Auftrennung auf diskontinuierliche oder kontinuierliche Art und Weise an Adsorptionsmitteln vom Typus stark saurer, mit Alkalimetall- oder Erdalkalimetallionen beladener kationischer Harze, oder vom Typus mit Ammonium-, Natrium-, Kalium-, Calcium-, Strontium- oder Bariumionen beladener Zeolithen durchgeführt wird.

5. Verfahren nach einem der Ansprüche 1 bis 4, dadurch gekennzeichnet, dass in der Stufe der chromatographischen Auftrennung mehrere Chromatographiesäulen zum Einsatz gelangen, welche mit Adsorptionsmitteln vom Typus starker kationischer Harze in der Calciumform und mit feiner Teilchengrösse gepackt sind, wobei eine Desorptionszone I, eine Adsorptionszone II und eine Zone III für die Anreicherung an den hydrierten Polyholosiden durch Stellung von Magnetventilen gebildet werden, wobei die letztgenannten auf Rohrleitungen angeordnet sind, welche den Ausgang irgendeiner beliebigen Säule mit dem Eingang der nächstfolgenden Säule verbinden, und wobei eine Verschlussvorrichtung eine vollständige Isolierung zwischen der Zone III, an deren Ende die hydrierten Polyholoside gewonnen werden, und der Zone I für die Desorption des Sorbits aufrecht erhält, wobei in den Kopf der letztgenannten Zone das Desorptionswasser eingeführt wird, und wobei die genannte Verschlussvorrichtung die Durchlaufrichtung der flüssigen Phase über das selektive Adsorptionsmittel gewährleistet und vor allem eine Verunreinigung des reinen Sorbits mit Spuren von Polyholosiden verhindert.

6. Verfahren nach einem der Ansprüche 1 bis 4, dadurch gekennzeichnet, dass die Stufe der sauren Hydrolyse der zweiten Fraktion über einem feststehenden sauren Katalysator durchgeführt wird, und dass sie derart vorgenommen wird, dass mehr als 40 Gew.-% der vorhandenen, hydrierten Disaccharide und Polysaccharide hydrolysiert werden, wobei die genannte Hydrolyse vorzugsweise kontinuierlich dadurch ausgeführt wird, dass man den die zu hydrolysierenden Polyole enthaltenden Sirup über ein Festbett führt, welches saure Katalysatoren vom Typus der Aluminosilikate, Kieselsäure, Aluminiumoxid oder kationischer Harze enthält.

7. Verfahren nach Anspruch 6, dadurch gekennzeichnet, dass die Hydrolyse über starken kationischen Harzen in Säureform bei einer zwischen 50 und 120 °C liegenden Temperatur durchgeführt wird, wobei die über das Bett aus saurem Katalysator geführte Durchsatzmenge derart geregelt wird, dass ein Hydrolysegrad der hydrierten Di- und Polysaccharide von wenigstens 60%, und vorzugsweise von wenigstens 80%, erhalten wird.

8. Verfahren nach einem der Ansprüche 1 bis 7, dadurch gekennzeichnet, dass der Gehalt an Trockensubstanz der zweiten Fraktion, welche der Stufe der Hydrolyse unterworfen wird und welche im Kreislauf in das Verfahren rückgeführt wird, im allgemeinen zwischen 1,5% und 30%, vorzugsweise zwischen 2,5% und 20% liegt.

9. Anlage zur Durchführung des Verfahrens nach einem der Ansprüche 1 bis 8, dadurch gekennzeichnet, dass sie umfasst:
— einen Behälter 201, in dessen Innerem die Stufe der katalytischen Hydrierung durchgeführt wird,
— eine Vorrichtung 202 für die chromatographische Auftrennung, und
— einen Reaktionsbehälter 203, in dessen Innerem die Stufe der Hydrolyse durchgeführt wird.

**Claims**

1. Process for the preparation of very high purity sorbitol syrup, characterized by the fact that a starting material of which a first part is constituted by a starch hydrolysate, is subjected to a process comprising in combination

— a catalytic hydrogenation step of the starting material,
— a chromatographic separation step of the starting material which has undergone hydrogenation into a first fraction containing very pure sorbitol which is recovered and into a second fraction containing, besides sorbitol, non-hydrogenated sugars as well as hydrogenated di- and polysaccharides,
— an acid hydrolysis step of the abovesaid second fraction, which provides a partially hydrogenated starch hydrolysate which is recirculated and which constitutes a second part of the starting material subjected to the hydrogenation step.

2. Process according to claim 1, characterized by the fact that the starch hydrolysate which is part of the constitution of the starting material shows, preferably, a true dextrose content comprised between 65 and 97% and, more preferably still, between 70 and 95%, particularly advantageous starch hydrolysates being constituted by hydrols of the first and second crop obtained during crystallization of the dextrose.

3. Process according to one of claims 1 and 2, characterized by the fact that the catalytic hydrogenation step is carried out on ruthenium or Raney nickel catalysts, preferably on a Raney nickel catalyst, at a hydrogen pressure comprised between 40 and 70 kg/cm$^2$ and at a temperature from about 100 to 150 °C.

4. Process according to one of claims 1 to 3, characterized by the fact that the chromatographic separation step is carried out discontinuously or continuously on adsorbants of the strongly acid cationic resin type, charged with alkaline or alkaline earth ions or of the zeolite type, charged with ammonium, sodium, potassium, calcium, strontium or barium ions.

5. Process according to one of claims 1 to 4, characterized by the fact that the chromatographic separation step employs several chromatography columns lined with adsorbant of the strong cationic resin type in the calcium form and of fine granulometry, a zone I of desorption, a zone II of adsorption and a zone III of enrichment of the hydrogenated polyholosides being formed by the setting of the electrovalves located in the pipes connecting the outlet of any one column to the inlet of the following column, a closure device maintaining total fluid-tightness between zone III, at the end of which are recovered the hydrogenated polyholosides, and zone I for desorption of the sorbitol, at the head of which zone the desorption water is introduced, said closure device ensuring the direction of the passage of the liquid phase over the selective adsorbant and avoiding particularly contamination of the pure sorbitol by traces of polyholosides.

6. Process according to one of claims 1 to 4, characterized by the fact that the acid hydrolysis step of the second fraction is carried out on a fixed acid catalyst and is conducted so that more than 40% by weight of the hydrogenated disaccharides and polysaccharides present are hydrolysed, said hydrolysis being effected continuously by passing the syrup containing the polyols to be hydrolysed over a fixed bed containing acid catalysts selected from the group consisting of the aluminosilicate, silica, alumina or cationic resin type.

7. Process according to claim 6, characterized by the fact that the hydrolysis is conducted on strong cationic resins in the acid form, at a temperature comprised between 50 and 120 °C, the passage flow rate over the acid catalytic bed being regulated so as to obtain a hydrolysis ratio of the hydrogenated di- and polysaccharides at least equal to 60% and, preferably, at least equal to 80%.

8. Process according to one of claims 1 to 7, characterized by the fact that the content of dry matter of the second fraction which is subjected to the hydrolysis step and which is recirculated is generally comprised between 1,5% and 30% and, preferably, between 2,5% and 20%.

9. Installation for carrying out the process according to one of claims 1 to 8, characterized by the fact that it comprises:

– a vessel 201 within which the catalytic hydrogenation step is carried out,

– a chromatographic separation installation 202 and

– a reaction vessel 203 within which the hydrolysis step is carried out.

1/4

FIGURE 1

Fig.2.

17

3/4

FIGURE 3

FIGURE 4